# DEMANDE DE BREVET EUROPEEN

(11) **EP 0 620 459 A2**
(43) Date de publication de la demande: **19.10.1994**
(21) Numéro de dépôt: 94400550.3
(22) Date de dépôt: 14.03.1994
(51) Int. Cl.: G02B 6/16, C03C 25/02

(54) **Procédé pour appliquer un revêtement réfléchissant sur une face frontale d'une fibre optique**

(30) Priorité: 15.03.1993 FR 9302953
(71) Demandeur: ALLIANCE TECHNIQUE INDUSTRIELLE, F-91080 Courcouronnes (FR)
(72) Inventeur: Malavieille, François-Louis, F-92290 Chatenay-Malabry (FR)
(74) Mandataire: Plaçais, Jean-Yves

(57) **Abrégé**

L'invention concerne un procédé pour appliquer un revêtement réfléchissant sur une face frontale d'une fibre optique, notamment d'une sonde médicale à fibre optique.

On soumet la face frontale (24) d'une fibre optique (10) à un flux contrôlé (45) de molécules-vapeur propres à former un dépôt multicouche diélectrique interférentiel dans des conditions telles que la face frontale (24) s'étende dans une direction sensiblement perpendiculaire à la direction générale du flux contrôlé (45), ce qui permet à la face frontale de présenter des couches interférentielles d'épaisseur contrôlée possédant des propriétés sélectives de miroir réfléchissant, tandis que les parois latérales de la fibre optique ne possèdent pas ces propriétés.

Application notamment aux sondes médicales.

## Description

L'invention concerne un procédé pour appliquer un revêtement réfléchissant sur une face frontale d'une fibre optique.

Elle concerne également une sonde médicale à fibre optique comprenant un tel revêtement réfléchissant, et utilisable à des fins de traitement ou de diagnostic d'un patient.

Il est connu d'appliquer un revêtement réfléchissant sur une face frontale d'une fibre optique de manière à former un miroir dirigé vers l'intérieur de la fibre optique.

Lorsque cette face frontale est oblique, un faisceau lumineux parcourant la fibre optique peut être réfléchi par le revêtement réfléchissant formant miroir de manière à sortir du coeur de la fibre et à traverser la gaine optique de celle-ci.

Dans les procédés connus, le revêtement réfléchissant est habituellement à base d'un dépôt métallique, par exemple d'aluminium, d'argent ou d'or, obtenu par évaporation sous vide.

L'application d'un tel dépôt métallique pose de nombreux problèmes pratiques, compte tenu notamment de la très petite taille des fibres optiques.

En outre, avec les procédés d'application connus, le revêtement réfléchissant métallique se trouve appliqué non seulement sur la face frontale de la fibre, mais aussi sur une partie de ses faces latérales qui sont situées à proximité, si bien que ces dernières sont également réfléchissantes à l'égard du faisceau lumineux introduit dans la fibre optique.

Or, dans les applications normales de ces fibres optiques, il faut que le faisceau lumineux réfléchi par le miroir puisse sortir du coeur de la fibre et traverser la gaine par une partie latérale, laquelle doit donc être transparente à l'égard de ce faisceau lumineux.

Dans les procédés connus, il a donc été nécessaire jusqu'à présent d'effectuer une opération supplémentaire pour enlever au moins en partie le revêtement réfléchissant formé sur les parties latérales de la fibre optique, de manière à rendre ces parties latérales transparentes.

Cette opération est délicate à réaliser compte tenu de la très petite taille des fibres optiques. Elle doit en outre être effectuée avec le plus grand soin pour ne pas endommager le miroir réfléchissant formé sur la face frontale et qui, lui, doit être conservé.

On connaît par ailleurs des sondes médicales à fibre optique qui comprennent une extrémité proximale propre à être connectée à une source de rayons lumineux et une extrémité distale propre à envoyer ces rayons lumineux dans une région avoisinante contenant un milieu biologique.

Ces sondes connues sont utilisées dans différentes applications médicales, par exemple pour explorer ou traiter des lésions ou tumeurs, notamment dans le cadre d'affections liées à la prostate chez l'homme.

La sonde est alors placée à l'intérieur d'un cathéter qui est introduit dans un canal ou une cavité du corps du patient, au travers soit d'un orifice naturel, soit d'une incision pratiquée par le chirurgien. En général, le cathéter sert aussi à faire passer un dispositif d'éclairage et de visualisation permettant au chirurgien de visualiser la zone explorée ou traitée, un outil tel que par exemple une pince de résection, un dispositif d'injection d'eau ou d'autre fluide, etc.

L'extrémité proximale de la sonde médicale est connectée à une source capable d'émettre des rayons lumineux du domaine visible et/ou du domaine invisible en fonction de l'application considérée.

Si les rayons lumineux à émettre sont invisibles, il convient de les doubler par des rayonnements lumineux visibles pour permettre au chirurgien de visualiser et contrôler l'émission de ces rayons lumineux invisibles.

Parmi les sources de rayons lumineux utilisables, on peut citer essentiellement les sources de lumière laser, comme les lasers YAG, les lasers à excimères, ou encore les lasers à CO₂. Toutefois, l'emploi de ces derniers pose des problèmes de transmission de longueur d'onde et nécessite des fibres optiques particulières.

Généralement, les rayons lumineux émis par la source ont une longueur d'onde comprise entre 280 et 2000 nanomètres, ce qui correspond au domaine allant du proche ultraviolet au proche infrarouge, et permet d'utiliser la plupart des types de fibres optiques, en particulier les fibres du type silice.

Les rayons lumineux émis par la source pénètrent dans la fibre par son extrémité proximale, se propagent ensuite axialement dans le coeur de la fibre pour quitter la fibre par son extrémité distale et illuminer une région du corps à explorer ou à traiter.

Les rayons lumineux ainsi dirigés sur cette région du corps, par exemple sur un tissu présentant une tumeur ou une lésion, peuvent traiter directement cette région par échauffement ou découpe. Ces rayons lumineux peuvent aussi être utilisés en photochimiothérapie pour agir sur certaines substances anticancéreuses, préalablement ingérées par le patient, qui ont la propriété d'être absorbées spécifiquement par les cellules cancéreuses ou tumorales. Lorsque de telles cellules sont illuminées par des rayonnements lumineux appropriés, les substances anticancéreuses libèrent de l'oxygène pour tuer ainsi les cellules ayant absorbé ces substances anticancéreuses.

Un des problèmes posés par ce type de sonde est celui de pouvoir contrôler l'orientation des rayons lumineux à la sortie de l'extrémité distale de la fibre optique.

En effet, les rayons lumineux quittent normalement la fibre optique dans la direction axiale de celle-ci, en formant un flux lumineux de forme conique dont l'angle au sommet dépend des caractéristiques de l'ouverture, dite "ouverture numérique", de la fibre optique.

Or, dans un certain nombre d'applications, et notamment celles où les sondes empruntent les voies naturelles du corps, les régions ou organes à traiter ne sont pas situés dans l'axe de la fibre, c'est-à-dire dans son prolongement, mais latéralement par rapport à celle-ci, c'est-à-dire à la périphérie des voies naturelles, par exemple la prostate chez l'homme, traversée par l'urètre.

Dans le but de dévier latéralement les rayons lumineux à la sortie de l'extrémité distale, on a proposé d'équiper cette extrémité distale, soit d'un embout diffusant, utilisé essentiellement en photochimiothérapie, soit encore d'une pièce métallique supportant un miroir disposé en regard et à distance de cette extrémité distale.

Ces embouts ou ces miroirs constituent des pièces volumineuses qui augmentent l'encombrement en largeur de la sonde et qui sont susceptibles de se salir au contact des tissus traités par les rayons lumineux et qui forment souvent des dépôts carbonisés empêchant l'embout ou le miroir d'exercer parfaitement sa fonction.

On a également proposé de former directement un miroir réfléchissant sur la face frontale d'une fibre optique comme enseigné par exemple par le document EP-A-0163266.

Toutefois, la réalisation d'un tel miroir réfléchissant conduit aux inconvénients mentionnés plus haut.

L'invention a notamment pour but de surmonter les inconvénients précités.

Sous un premier aspect, l'invention propose un procédé pour appliquer un revêtement réfléchissant sur une face frontale d'une fibre optique, ce procédé étant caractérisé en que l'on soumet la face frontale à un flux contrôlé de molécules-vapeur propre à former un dépôt multicouche diélectrique interférentiel par évaporation sous vide, dans des conditions telles que la face frontale s'étende dans une direction sensiblement perpendiculaire à la direction générale du flux contrôlé, ce qui permet à la face frontale de présenter des couches interférentielles d'épaisseur contrôlée possédant des propriétés sélectives de miroir réfléchissant, tandis que les parois latérales de la fibre optique ne possèdent pas ces propriétés.

Il en résulte que la face frontale ainsi traitée devient réfléchissante pour une longueur d'onde de travail donnée, tandis que les parois latérales de la fibre restent transparentes à cette longueur d'onde.

Les parois latérales reçoivent en effet des dépôts d'épaisseur différente qui sont par conséquent transparents à cette longueur d'onde.

De ce fait, il n'est plus nécessaire de prévoir une opération supplémentaire de traitement des faces latérales de la fibre, comme exigé avec les procédés de la technique antérieure dans lesquels le revêtement réfléchissant est un dépôt métallique.

Le revêtement réfléchissant de l'invention est composé de couches alternées d'indices haut et bas, dont l'épaisseur est sensiblement égale au quart de la longueur d'onde du faisceau lumineux à réfléchir.

Avantageusement, la face frontale est formée obliquement par rapport à l'axe de la fibre optique.

Selon une autre caractéristique avantageuse, on applique le revêtement réfléchissant simultanément sur les faces frontales respectives d'une multiplicité de fibres optiques disposées en faisceau.

Sous un autre aspect, l'invention concerne une sonde médicale à fibre optique du type défini en introduction, dans laquelle l'extrémité distale, dénudée localement de sa gaine mécanique, comprend une face frontale oblique munie d'un revêtement réfléchissant formant un miroir dirigé du côté de la fibre, caractérisée en ce que le revêtement réfléchissant comprend un dépôt multicouche diélectrique interférentiel d'épaisseur contrôlée appliqué par évaporation sous vide, de telle sorte que le miroir réfléchisse les rayons lumineux pour les faire sortir du coeur de la fibre vers sa gaine optique, les rayons lumineux traversant la gaine optique de la fibre, par une partie latérale de celle-ci, pour atteindre la région avoisinante.

L'invention permet ainsi de former directement un miroir sélectif sur une face frontale oblique de l'extrémité distale.

Les rayons lumineux guidés par le coeur de la fibre sont déviés latéralement d'un angle supérieur à l'angle limite de guidage du coeur optique et traversent ainsi la gaine optique par la partie latérale préalablement dénudée de toute protection mécanique.

Par l'expression "face frontale oblique", on veut dire ici que cette face s'étend dans un plan incliné par rapport à l'axe de la fibre optique.

Le revêtement réfléchissant appliqué sur cette face frontale oblique forme un miroir dirigé du côté de la fibre, c'est-à-dire vers l'intérieur de celle-ci, et donc complètement protégé vis-à-vis de sources de salissures extérieures.

Les rayons lumineux se propageant dans le coeur de la fibre sont réfléchis par le miroir et déviés vers une partie latérale de la fibre.

De façon surprenante, on a constaté que si l'extrémité de la fibre portant le miroir était plongée dans un liquide transparent ou translucide tel de l'eau, les rayons lumineux sortant par la paroi latérale cylindrique dénudée ne subissaient aucune perturbation notable en divergence ou en puissance, par rapport aux caractéristiques de sortie naturelle axiale de la fibre. Le liquide, par son indice de réfraction très proche de celui de la silice de la fibre, annulant le dioptre cylindrique latéral, dioptre cylindrique latéral qui, dans l'air, provoque une déviation et une divergence importante des rayons réfléchis par le miroir. Or, la plupart des voies naturelles du corps sont remplies de liquides aqueux, ou peuvent être irriguées artificiellement par injection d'eau ou autre liquide.

En complément, ou en variante, on polit une petite surface plane en regard du miroir, dans la zone de sortie des rayons lumineux. Ceci peut permettre de s'affranchir de la nécessité de l'immersion pour éviter la perturbation du dioptre courbe de la partie latérale de la fibre, lorsque l'application de la sonde le nécessite.

Les rayons lumineux sont ainsi déviés latéralement dans une direction que l'on peut contrôler d'une part par construction de la fibre, en choisissant l'angle d'inclinaison de la face frontale, d'autre part in situ, par rotation et translation de la fibre elle-même pendant l'opération.

Dans une forme de réalisation préférée de l'invention, cette face frontale est plane et forme, avec l'axe de la fibre optique, un angle compris entre 20 et 60°.

De préférence, cet angle est égal à 45° environ, ce qui permet de dévier les rayons lumineux dans une direction généralement perpendiculaire à l'axe de la fibre optique.

Dans une variante de réalisation, la face frontale de l'extrémité distale présente une surface convexe sphérique, ce qui permet de former un miroir concave et, par conséquent, de réaliser une sorte de focalisation des rayons lumineux sortant latéralement de l'extrémité distale.

D'autres caractéristiques et avantages de l'invention apparaîtront à l'examen de la description et des dessins annexés, sur lesquels :
- la figure 1 est une représentation schématique d'une sonde médicale à fibre optique selon l'invention;
- la figure 2 représente un faisceau de fibres dont les extrémités respectives subissent une opération de polissage sur un disque;
- la figure 3 est une vue partielle en perspective des extrémités respectives des fibres du faisceau, après polissage;
- la figure 4 représente schématiquement une enceinte pour le dépôt d'un revêtement réfléchissant sur les extrémités des fibres;
- la figure 5 représente une bride pour supporter les fibres dans l'enceinte au-dessus d'un creuset;
- la figure 6 représente, à échelle agrandie, l'extrémité d'une fibre munie d'un dépôt réfléchissant;
- la figure 7 représente l'extrémité distale d'une fibre dans une variante de réalisation;
- la figure 8 représente l'extrémité distale d'une fibre dans une autre variante de réalisation;
- la figure 9 est une vue latérale d'une coiffe de protection adapté sur l'extrémité distale d'une fibre; et
- la figure 10 est une vue de face de la coiffe de protection.

La technique des fibres optiques fait à de nombreux titres intervenir des formes. A cet égard, les dessins annexés sont à considérer comme faisant partie intégrante de la présente description, pour contribuer autant que nécessaire à la définition de l'invention et à la suffisance de la description de celle-ci.

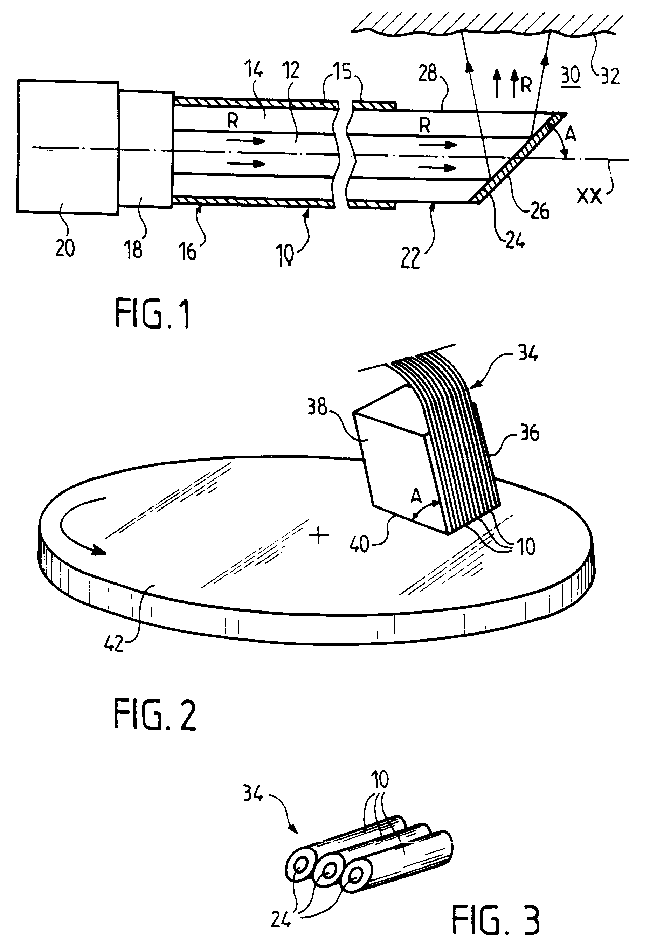

On se réfère tout d'abord à la figure 1 qui représente une sonde médicale qui comprend une fibre optique 10, constituée, de façon en soi connue, d'un coeur optique 12, entouré d'une gaine optique 14, elle-même entourée d'une gaine polymère de protection mécanique 15.

La fibre optique 10 possède une extrémité proximale 16 reliée, au travers d'un connecteur 18, à une source 20 propre à émettre des rayons lumineux dans le domaine visible et/ou invisible. La source 20 peut être, par exemple, une source de lumière laser comme indiqué plus haut.

La source 20 envoie ainsi, dans le coeur 12 de la fibre, des rayons lumineux R qui se propagent dans la direction de l'axe XX de la fibre.

La fibre optique 10 possède une extrémité distale 22 comportant une face frontale oblique 24. Dans l'exemple, il s'agit d'une face plane faisant un angle A de 45° par rapport à l'axe XX. La face frontale 24 est polie et reçoit un revêtement réfléchissant 26 formant un miroir dirigé du côté de la fibre, c'est-à-dire vers l'intérieur de celle-ci. Au voisinage de cette face 24, la fibre optique est dénudée, c'est-à-dire débarrassée de sa gaine mécanique 15.

Les rayons lumineux R sont de ce fait réfléchis par le miroir et déviés à 90° vers une partie latérale dénudée 28 de la fibre.

Les rayons ainsi déviés traversent ensuite la partie latérale 28 de la gaine optique, et sortent de la fibre sans perturbation notable du fait de l'annulation du dioptre d'interface par le liquide 30 se trouvant dans le voisinage de l'extrémité distale 22 préalablement introduite dans un canal ou une cavité naturel d'un patient.

Comme on le voit sur la figure 1, la partie latérale 28 de l'extrémité distale 22 se trouve en regard d'une région 32 qui peut consister, par exemple, en un tissu présentant une lésion ou une tumeur.

La sonde de l'invention permet ainsi de diriger le rayon lumineux directement sur la région 32, dans une direction générale perpendiculaire à l'axe XX de la fibre.

Pour réaliser la face frontale oblique à l'extrémité distale de la fibre, on peut procéder de la manière décrite dans le Brevet français No 87 01952 déposé au nom de la Demanderesse. Cette technique a été utilisée dans un domaine totalement différent pour réaliser des coupleurs multiplexeurs/démultiplexeurs.

Cette technique permet de réaliser simultanément des faces frontales obliques sur les différentes fibres d'un faisceau 34.

Ensuite, comme montré à la figure 2, une extrémité du faisceau 34 est positionnée sur une face inclinée 36 d'une cale 38 dont une face inférieure 40 est maintenue en appui sur une surface de polissage d'un disque de polissage 42. Les faces 36 et 40 de la cale 38 font entre elles un angle A correspondant à la valeur souhaitée pour les faces frontales obliques des fibres 10 du faisceau 34. Cette technique de polissage permet de former, pour chacune des fibres 10, une face frontale oblique parfaitement polie et dont l'angle A peut être déterminé avec une précision de 0,1°.

Le polissage des extrémités des fibres s'effectue en exerçant un effort d'appui de la cale 38 en direction du plateau 42. Avantageusement, cette opération d'usinage se fait en commençant par un disque à grains abrasifs grossiers et en terminant par un disque à grains abrasifs fins.

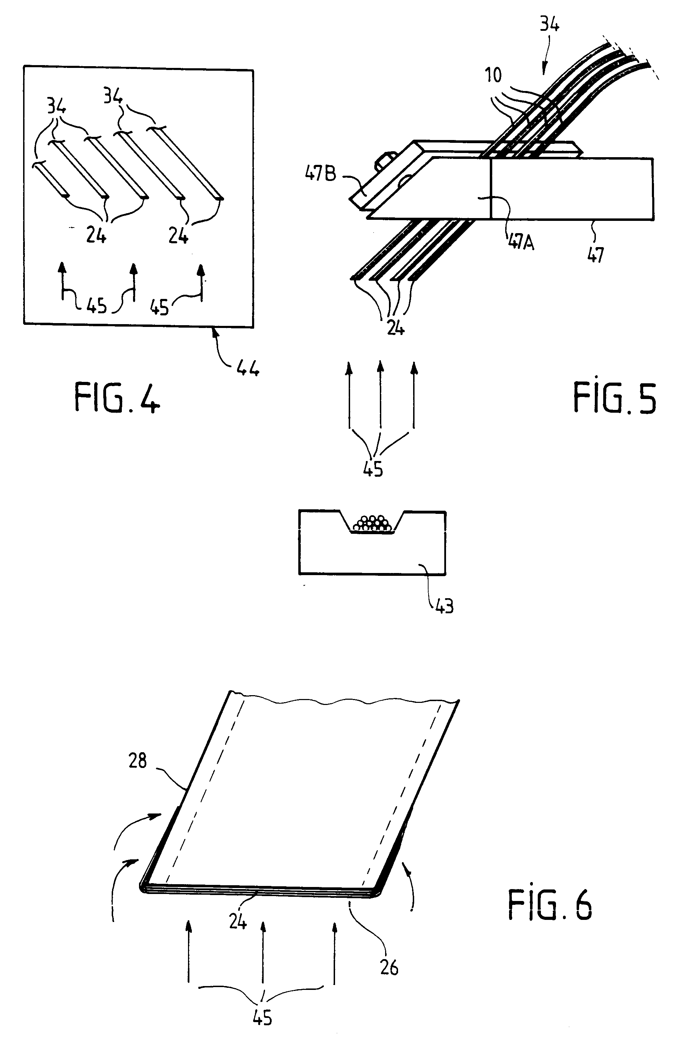

Lorsque l'opération de polissage est terminée, toutes les fibres 10 du faisceau 34 présentent, comme montré à la figure 3, une face frontale oblique 24 non perpendiculaire à l'axe de la fibre.

Le revêtement réfléchissant est obtenu par évaporation sous vide d'un dépôt multicouche diélectrique interférentiel. Ce miroir est ainsi composé de couches alternées d'indice haut et bas, tandis que l'épaisseur des couches est sensiblement égale au quart de la longueur d'onde du faisceau lumineux à réfléchir.

Le grand avantage de cette réalisation est sa capacité à réfléchir sans dommage les très fortes puissances optiques comme celles de lasers YAG par exemple.

On décrira maintenant le procédé d'application du revêtement réfléchissant par évaporation sous vide d'un dépôt multicouche diélectrique interférentiel.

Dans une opération ultérieure (figure 4), on utilise une enceinte 44, maintenue sous vide et comprenant un creuset d'évaporation 43 (figure 5) définissant un flux 45 de molécules-vapeur. L'enceinte 44 est du type utilisé en fabrication de miroirs dichroïques, ou lames semi-réfléchissantes, sur lesquelles on effectue des dépôts de différents matériaux d'indices optiques alternés. On peut appliquer des couches, dites "couches interférentielles", possédant des propriétés sélectives permettant à une longueur d'onde donnée de traverser le miroir par transmission, et à une autre longueur d'onde donnée d'être réfléchie par ce miroir.

Pour les fibres optiques, cette enceinte est utilisée d'une manière particulière.

On dispose plusieurs faisceaux 34 de fibres 10 dans l'enceinte 44, de telle sorte que les faces frontales respectives 24 des fibres s'étendent dans un plan sensiblement perpendiculaire à la direction générale du flux de molécules s'échappant du creuset 43 à l'état de vapeur (figure 5).

On utilise pour cela une bride support 47 disposée au-dessus du creuset 43 et possédant deux mâchoires 47A et 47B entre lesquelles un ou plusieurs faisceaux 34 sont maintenus dans la position désirée.

Du fait que les parois latérales 28 dénudées sont orientées presque parallèlement au flux 45 de molécules-vapeur (figure 6), elles reçoivent des dépôts d'épaisseurs très différentes de celles de la face frontale 24 de la fibre (dont l'oblicité est ramenée perpendiculairement au flux de vapeur).

On obtient ainsi, sur la face frontale 26, des couches dont l'épaisseur est contrôlée et correspond au quart de la longueur d'onde et font donc miroir à cette longueur d'onde. Par contre, dans les parties latérales de la fibre, où l'épaisseur des couches est inférieure au quart de la longueur d'onde par effet de masquage naturel, le dépôt ainsi obtenu est transparent à cette longueur d'onde.

L'appareil de dépôt sous vide étant réglé pour que la face frontale soit réfléchissante pour la longueur d'onde de travail, par contre les parois latérales dénudées de la fibre restent transparentes à la longueur d'onde de travail. Ceci est particulièrement important car, du fait de la très petite taille des fibres optiques, il n'existe que très peu de techniques susceptibles d'y réaliser des dépôts de ce genre à l'échelle industrielle.

De cette manière, on peut déposer sélectivement sur la face frontale de la fibre plusieurs couches ayant chacune une épaisseur contrôlée correspondant au quart de la longueur d'onde désirée.

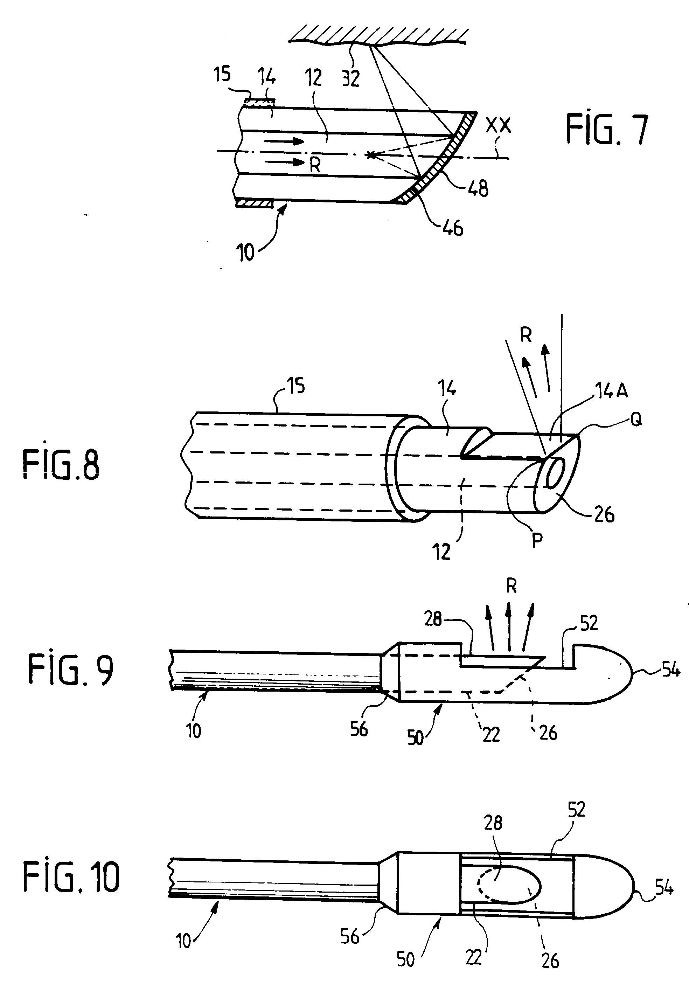

Dans la variante représentée à la figure 7, l'extrémité distale de la fibre 22 possède une face frontale oblique 46 qui est de forme convexe et sphérique. Cette face convexe peut être obtenue par une technique de polissage analogue à celle décrite plus haut en référence à la figure 2, mais en utilisant un disque de polissage mou au lieu d'un disque de polissage dur.

Sur la face frontale 46 est appliqué un revêtement réfléchissant 48 analogue au revêtement 26 décrit précédemment. Le revêtement 48 permet ainsi de former un miroir concave pour focaliser les rayons lumineux R sur la région 32 à explorer ou traiter.

Dans la variante représentée à la figure 8, au voisinage de l'extrémité distale de la fibre 22, il est prévu un polissage latéral 14A de la gaine optique de la fibre. Ce polissage peut être obtenu par une technique analogue à celle décrite plus haut en référence à la figure 2. Il est de préférence plan (en tout cas moins courbe que la surface externe de la gaine optique). Le plan de la face polie 14A peut être parallèle à l'axe de la fibre. Le segment PQ obtenu par intersection de la face polie 14A et de la face frontale oblique 26 peut vérifier la propriété suivante de symétrie : le plan médiateur du segment PQ passe sensiblement par l'axe de la fibre. Toutefois, d'autres inclinaisons et d'autres symétries peuvent être envisagées.

Par contre, il est estimé important, actuellement, que le polissage de la face 14A reste dans la gaine optique de la fibre, sans atteindre le coeur de celle-ci.

Dans la forme de réalisation représentée aux figures 9 et 10, l'extrémité distale 22 de la fibre 10 est munie d'une coiffe de protection 50 présentant une fenêtre latérale 52 placée en regard de la partie latérale 28 (ou 14A) de la fibre qui est traversée par les rayons lumineux R.

La coiffe de protection 50 est formée d'un tube cylindrique creux réalisé en matériau opaque, par exemple en matériau métallique, le tube étant muni d'une extrémité fermée arrondie 54. La coiffe est emmanchée sur l'extrémité distale 22 de la fibre et est maintenue dans la position désirée, en laquelle la fenêtre latérale 52 est placée en regard de la partie latérale de la fibre, en réalisant un collage ou sertissage de l'autre extrémité 56 du tube cylindrique.

La coiffe de protection 50 constitue un élément de sécurité dans le cas où le revêtement réfléchissant 26 (ou 48) serait endommagé accidentellement, ce qui entraînerait une sortie des rayons lumineux R dans l'axe de la fibre.

En variante, la fenêtre 52 de la coiffe 50 pourrait être fermée par un élément transparent en verre. Le cas échéant, cet élément pourrait servir à retenir de l'eau dans la coiffe autour de l'extrémité distale de la fibre optique.

Il est à noter que la coiffe de protection 50 augmente très peu l'encombrement latéral de la fibre et qu'elle facilite en outre l'introduction de la fibre du fait de son extrémité arrondie 54.

Le procédé de l'invention permet d'appliquer un revêtement réfléchissant sur une face frontale, oblique ou non, d'une fibre optique.

De façon préférentielle, l'invention s'applique à une sonde médicale utilisable dans le diagnostic et le traitement de différentes affections, notamment des affections liées à des lésions et tumeurs.

Elle peut servir notamment dans les voies naturelles, de préférence avec une fibre dont la gaine optique est mince. Par contre, lorsqu'un polissage latéral 14A est requis, on préférera une fibre à gaine optique épaisse.

Pour une action médicale donnée, on pourra disposer de plusieurs sondes (ou de plusieurs cathéters incorporant chacun au moins une sonde), différant entre elles par les caractéristiques de leur faisceau optique de sortie, dues notamment à l'inclinaison de la face 26, à sa forme concave, plane, ou autre, ainsi qu'à la taille de la fibre, et à son éventuel polissage 14A. In situ, par rotation et/ou translation de la fibre elle-même pendant l'opération, on détermine avec précision la zone illuminée.

Ceci convient pour les opérations de la prostate chez l'homme, traversée par l'urètre. Mais l'invention est d'application beaucoup plus générale, non seulement comme sonde empruntant les voies naturelles du corps, mais aussi partout où une illumination intense et/ou contrôlée et/ou latérale est souhaitée, comme c'est le cas en photochimiothérapie.

## Revendications

**1.-** Procédé pour appliquer un revêtement réfléchissant sur une face frontale d'une fibre optique,
caractérisé en ce que l'on soumet la face frontale (24 ;46) à un flux contrôlé (45) de molécules-vapeur propres à former un dépôt multicouche diélectrique interférentiel par évaporation sous vide, dans des conditions telles que la face frontale s'étende dans une direction sensiblement perpendiculaire à la direction générale du flux contrôlé, ce qui permet à la face frontale de présenter des couches interférentielles d'épaisseur contrôlée possédant des propriétés sélectives de miroir réfléchissant, tandis que les parois latérales (28) de la fibre optique ne possèdent pas ces propriétés.

**2.-** Procédé selon la revendication 1, caractérisé en ce que la face frontale (24 ;46) est formée obliquement par rapport à l'axe de la fibre optique.

**3.-** Procédé selon l'une des revendications 1 à 2, caractérisé en ce que l'on applique le revêtement réfléchissant (26;48) simultanément sur les faces frontales respectives (24;46) d'une multiplicité de fibres optiques disposées en faisceaux.

**4.-** Sonde médicale à fibre optique, du type comprenant une extrémité proximale (16) propre à être connectée à une source (20) de rayons lumineux (R) et une extrémité distale (22) propre à envoyer ces rayons lumineux dans une région avoisinante, et dans laquelle l'extrémité distale (22), dénudée localement de sa gaine mécanique (15), comprend une face frontale oblique (24;46) munie d'un revêtement réfléchissant (26;48) formant un miroir dirigé du côté de la fibre, caractérisée en ce que le revêtement réfléchissant (26;48) comprend un dépôt multicouche diélectrique interférentiel d'épaisseur contrôlée appliqué par évaporation sous vide, de telle sorte que le miroir réfléchisse les rayons lumineux (R) pour les faire sortir du coeur (12) de la fibre vers sa gaine optique (14), les rayons lumineux (R) traversant la gaine optique (14) de la fibre par une partie latérale (28;14A) de celle-ci pour atteindre ladite région avoisinante.

**5.-** Sonde médicale selon la revendication 4, caractérisée en ce que, ladite région avoisinante contenant un liquide aqueux (30), les rayons lumineux (R) sortent de la gaine optique (14) sans perturbation, du fait de l'interface sans dioptre existant entre cette gaine optique (14) et le milieu liquide (30).

**6.-** Sonde médicale selon l'une des revendications 4 et 5, caractérisée en ce que la partie latérale (14A) de la gaine optique (14) d'où sortent les rayons lumineux (R) est localement polie selon une surface plane.

**7.-** Sonde médicale selon l'une des revendications 4 à 6, caractérisée en ce que la direction générale de la face frontale (24) de l'extrémité distale (22) forme, avec l'axe (XX) de la fibre optique (10), un angle (A) compris entre 20 et 60°.

**8.-** Sonde médicale selon la revendication 7, caractérisée en ce que l'angle (A) formé entre la face frontale (24) et l'axe (XX) de la fibre (10) est d'environ 45°.

**9.-** Sonde médicale selon l'une des revendications 4 à 8, caractérisée en ce que la face frontale (24) de l'extrémité distale (22) est plane.

**10.-** Sonde médicale selon l'une des revendications 4 à 8, caractérisée en ce que la face frontale (46) de l'extrémité distale (22) de la fibre présente une surface convexe sphérique, ce qui permet de former un miroir concave.
